# EUROPÄISCHE PATENTSCHRIFT

(11) **EP 1 849 493 B1**
(45) Veröffentlichungstag und Bekanntmachung des Hinweises auf die Patenterteilung: **06.01.2010**
(21) Anmeldenummer: 07006051.2
(22) Anmeldetag: 23.03.2007
(51) Int. Cl.: A61M 25/00

(54) **Katheterset für die epidurale oder periphere Nervenblockade**
Catheter set for epidural or peripheral nerve block
Appareillage de cathéter pour le blocage des nerfs épidural ou périphérique

(30) Priorität: 28.04.2006 DE 102006020363
(43) Veröffentlichungstag der Anmeldung: 31.10.2007
(73) Patentinhaber: Pajunk GmbH & Co. KG Besitzverwaltung, 78187 Geisingen (DE)
(72) Erfinder: Pajunk, Heinrich, 78187 Geisingen (DE); Pajunk, Horst, 78187 Geisingen (DE); Tsui, Ban C.H., Edmonton Alberta T6G 2G3 (CA)
(74) Vertreter: Mussgnug, Bernd

(56) Entgegenhaltungen:
- WO-A-02/055145
- AT-B- 406 121
- DE-A1- 19 807 487
- US-A- 5 807 324

## Beschreibung

Die Erfindung betrifft ein Katheterset für die epidurale oder periphere Nervenblockade gemäß dem Oberbegriff des Anspruchs 1.

Bei der epiduralen Nervenblockade wird ein Anästhetikum in den Epiduralraum injiziert, um die tieferliegenden Körperpartien des Patienten zu betäuben. Dabei wird mit einer Kanüle in den Wirbelkanal eingestochen, über diese Kanüle wird ein Katheter eingeführt, der in dem Epiduralraum bis in den Bereich vorgeschoben wird, in welchem das Anästhetikum appliziert werden soll. Insbesondere bei einer Thorax-Nervenblockade kann dabei im kaudalen oder lumbalen Wirbelbereich eingestochen werden, um den Katheter über eine relativ große Länge bis in den Brustwirbel-Bereich zu schieben.

Bei der peripheren Nervenblockade, wie sie insbesondere für die operative oder analgetische Behandlung der Extremitäten angewendet wird, wird mit einer Kanüle in die Nervenscheide eingestochen und ein durch die Kanüle zugeführter Katheter wird in der Nervenscheide entlang des Nervs bis in den Bereich vorgeschoben, in welchem das Anästhetikum appliziert werden soll.

Aus der DE 101 00 976 C2, veröffentlicht auch als WO 02/055145 A1, ist ein Katheter für die Nervenblockade bekannt, der mittels Elektrostimulation positioniert werden kann. Hierzu weist der flexible elektrisch isolierende Kunststoff-Katheter an seinem distalen Ende eine blank liegende elektrisch leitende Stimulationsspitze auf, die über eine im Inneren des Katheters verlaufende leitende Verbindung mit einem am proximalen Ende des Katheters angebrachten Stimulationsanschluss verbunden ist. Ein an den Stimulationsanschluss angeschlossenes Stimulationsgerät schickt elektrische Stimulationspulse zu der distalen Stimulationsspitze, um den Nerv im Bereich dieser Stimulationsspitze zu stimulieren und dadurch dem Anästhesisten die Position der distalen Katheterspitze anzuzeigen. Der Katheter muss einerseits eine ausreichende Flexibilität aufweisen, um beim Vorschub den Nervenstrukturen ausweichen zu können, damit keine Schädigung des Nervs eintritt. Andererseits kann eine solche Flexibilität nachteilig sein, wenn der Katheter über eine größere Distanz vorgeschoben werden muss. In Extremsituationen kann sich das flexible distale Ende des Katheters beim Auftreffen auf einen Widerstand nach hinten umbiegen.

Aus der US 5 976 110 A ist ein Katheterset für die periphere Nervenblockade bekannt, bei welcher die Kanüle für den Einstich in die Nervenscheide eine distale Stimulationsspitze aufweist, mit welcher die exakte Position der Kanülenspitze in der Nervenscheide feststellbar ist. Ein flexibler Katheter wird in diese Kanüle soweit eingeführt, dass seine distale Spitze mit der distalen Stimulationsspitze der Kanüle übereinstimmt. Wird dann die Kanüle abgezogen, so befindet sich die distale Spitze des Katheters in der mittels der Stimulationskanüle ermittelten Position. Der Katheter kann koaxial durch einen auf das proximale Ende der Kanüle aufsetzbaren Schlauchadapter zugeführt werden. Das distale Ende des Katheters kann nur in Übereinstimmung mit dem distalen Ende der Kanüle positioniert werden, ein Vorschieben des Katheters über das distale Ende der Kanüle hinaus, wie es insbesondere für die Epiduralanästhesie notwendig ist, ist nicht vorgesehen.

Aus der EP 1 002 500 A1 ist ein Katheterset für die Plexusanästhesie bekannt, bei welchem mittels einer Stimulationskanüle eine Kunststoff-Verweilkanüle in die Nervenscheide eingesetzt wird, durch welche ein Katheter eingeführt werden kann. In dem flexiblen Kunststoff-Katheter liegt ein Stimulationsdraht, der am distalen Ende des Katheters über Öffnungen des Katheters freiliegt und an seinem proximalen Ende an einen Stimulator angeschlossen wird. Der Stimulationsdraht bewirkt eine Versteifung des flexiblen Kunststoff-Katheters. Der Stimulationsdraht muss dabei während des Vorschiebens des Katheters stets bis in dessen distale Spitze geführt sein, da der Stimulationsdraht an dieser distale Spitze die elektrische Stimulation des Nervs für die Positionierung bewirken muss. Der Katheter verliert durch diesen Stimulationsdraht die Flexibilität, die für ein risikoloses Vorschieben der Katheterspitze erwünscht ist.

Der Erfindung liegt die Aufgabe zugrunde, ein Katheterset für die epidurale oder periphere Nervenblockade zur Verfügung zu stellen, welches ein exaktes Positionieren der distalen Katheterspitze und ein Vorschieben des Katheters mit möglichst geringem Risiko auch über eine längere Distanz vereinigt.

Diese Aufgabe wird erfindungsgemäß gelöst durch ein Katheterset mit den Merkmalen des Patentanspruchs 1.

Vorteilhafte Ausführungen der Erfindung sind in den Unteransprüchen angegeben.

Das erfindungsgemäße Katheterset weist eine Kanüle auf, die zum Einstich in den Epiduralraum bzw. in die periphere Nervenscheide dient. Diese Kanüle kann zweckmäßig in an sich bekannter Weise als unipolare Stimulationskanüle ausgebildet sein. Eine solche Stimulationskanüle ist vorzugsweise als Stahlkanüle ausgebildet, die an ihrem Außenmantel elektrisch isolierend beschichtet ist, sodass nur ihre distale spitze in einem punktförmigen Bereich frei bleibt. Über einen proximalen Stimulationsanschluss kann die exakte Position dieser distalen Kanülenspitze im Epiduralraum bzw. in der peripheren Nervenscheide lokalisiert werden. Die distale Spitze der Kanüle kann in ebenfalls bekannter Weise mit einem Facettenschliff oder als Tuohy-Spitze oder als Sprotte-Spitze ausgebildet sein.

Durch die Kanüle wird ein Katheter in den Epiduralraum bzw. die Nervenscheide eingeschoben, der als flexibler nichtleitender Katheter, vorzugsweise aus Kunststoff, ausgebildet ist. Dabei kann der Katheter als vollständig aus Kunststoff bestehender Schlauch ausgebildet sein. Es können auch Katheter verwendet werden, bei welchen ein schraubenlinienförmig gewickelter dünner Draht in den Kunststoffschlauch eingelegt ist, um die Knickfestigkeit des Katheters zu verbessern, ohne dessen Biegsamkeit zu beeinträchtigen. Der Katheter dient zum Zuführen eines flüssigen Anästhetikums, welches über einen Spritzenanschluss am proximalen Ende in den Katheter eingeleitet wird und über wenigstens eine Austrittsöffnung am distalen Ende des Katheters austritt. Um das distale Ende des Katheters mit der Austrittsöffnung exakt positionieren zu können, ist der Katheter als Stimulationskatheter ausgebildet. Hierzu weist der Katheter an seinem distalen Ende eine freiliegende elektrisch leitende Stimulationsspitze auf, die über eine im Inneren des Katheters verlaufende elektrisch leitende Verbindung, z. B. einen dünnen Draht, mit einem am proximalen Ende des Katheters angeordneten elektrischen Stimulationsanschluss verbunden ist. Die leitende Verbindung ist dabei so ausgebildet, dass sie das innere Lumen des Katheters größtmöglich freilässt. Ist die leitende Verbindung z. B. als Draht ausgebildet, so ist der Durchmesser dieses Drahtes wesentlich kleiner als der freie Innendurchmesser des Katheters. Die Austrittsöffnung am distalen Ende des Katheters kann axial durch die distale Stirnfläche des Katheters verlaufen, sodass die zugeführte Flüssigkeit in der Vorschubrichtung des Katheters nach vorn austritt. Eine solche Austrittsrichtung ist z. B. bei der peripheren Nervenblockade bevorzugt. Die Austrittsöffnung kann auch am distalen Ende des Katheters seitlich in der Katheterwandung angeordnet sein, sodass sich eine radiale Austrittsrichtung der Flüssigkeit im Wesentlichen senkrecht zur Katheterachse ergibt. Eine solche Austrittsrichtung wird teilweise bei der epiduralen Nervenblockade bevorzugt. Es können auch zwei oder mehr Austrittsöffnungen vorgesehen sein, wobei eine axiale und eine oder mehrere radiale Austrittsöffnungen kombiniert werden können. Ein Stimulationskatheter dieser Art ist im wesentlichen aus der DE 101 00 976 C2 bekannt.

Erfindungsgemäß wird in diesen flexiblen Katheter ein Versteifungsmandrin eingeschoben, der nur zur Versteifung des flexiblen Katheters dient und nicht zur elektrischen Stimulation. Der Versteifungsmandrin kann maximal bis in die distale Spitze des Katheters vorgeschoben werden, um dadurch den Katheter bis in seine distale Spitze hinein zu versteifen. Dadurch wird ein Vorschub des Katheters auch über eine größere Distanz erleichtert. Der Versteifungsmandrin wird während des Vorschubs des Katheters variabel von der distalen Spitze des Katheters zurückgezogen, sodass vor der distalen Spitze des Versteifungsmandrins ein unversteifter flexibler Endbereich des Katheters verbleibt. Dieser flexible distale Spitzenbereich des Katheters erlaubt während des Vorschiebens ein Ausweichen der Katheterspitze beim Auftreffen auf einen Widerstand, sodass eine Schädigung insbesondere von Nervengewebe oder eine Dura-Perforation durch die Katheterspitze beim Vorschub weitestgehend ausgeschlossen ist. Durch die variable axiale Verschiebung des Versteifungsmandrins in dem Katheter kann die Länge der freien nachgiebig-flexiblen Katheterspitze während des Vorschiebens gewählt und den anatomischen Gegebenheiten fortlaufend angepasst werden.

Das Katheterset vereinigt dadurch eine optimale Steifigkeit des Katheters über nahezu dessen gesamte Länge für den Vorschub mit einer Flexibilität der distalen Spitze des Katheters zur Vermeidung von Schädigungen.

In einer vorteilhaften Ausführung weist der Katheter an seinem proximalen Ende einen Anschlussadapter auf, der einen seitlichen Stimulationsanschluss für den Anschluss des elektrischen Stimulators aufweist und die koaxiale Einführung des Versteifungsmandrins in das proximale Ende des Katheters ermöglicht.

Weiter ist zweckmäßig am proximalen Ende des Katheters ein Schlauchadapter ansetzbar, der über einen seitlich einmündenden Zuspritzschlauch das Anschließen einer Spritze an den Katheter ermöglicht, während der Versteifungsmandrin koaxial auch durch den Schlauchadapter einführbar ist. Der Schlauchadapter ist insbesondere mit einem hämostatischen Ventil ausgebildet. Über den Zuspritzschlauch kann insbesondere während des Einführens des Katheters einer Flüssigkeit zugespritzt werden. Dies kann ein Anästhetikum sein. Insbesondere kann aber auch eine Flüssigkeit zugespritzt werden, die den Nervenkanal bzw. den Epiduralraum aufweitet und ein schwimmendes Gleiten der Katheterspitze beim Vorschub erleichtert. Eine solche Flüssigkeit ist beispielsweise eine 5%-ige Lösung von Dextrose in Wasser. Diese Flüssigkeit hat die dilatierende und aufschwimmende Funktion, ohne die elektrische Stimulation der Stimulationsspitze zu beeinflussen. Die Dilatation durch die Flüssigkeit kann dabei auch zu einer zusätzlichen Lokalisierung der Katheterspitze mittels Ultraschall verwendet werden. Es ist auch möglich, eine physiologische Kochsalzlösung als Flüssigkeit zuzuspritzen, um durch die Leitfähigkeit der Kochsalz-Lösung die elektrische Nervenstimulation durch die Kathaterspitze zu unterstützen. Das hämostatische Ventil des Schlauchadapters ermöglicht auch eine Aspiration mittels der Spritze, um die Lage der Katheterspitze zu überprüfen und z. B. eine Perforation von Blutgefäßen zu vermeiden.

Vorzugsweise ist die distale Spitze des Katheters geschlossen bis auf die gegebenenfalls in der Katheterspitze axial vorgesehene Austrittsöffnung. Dadurch ist auch zusätzlich sichergestellt, dass der Versteifungsmandrin nicht distal aus dem Katheter austreten kann.

Im Folgenden wird die Erfindung anhand eines in der Zeichnung dargestellten Ausführungsbeispiels näher erläutert. Es zeigen:
- Figur 1: eine Kanüle für das Katheterset,
- Figur 2: das Katheterset gemäß der Erfindung und
- Figur 3: in vergrößerter Darstellung einen Axialschnitt durch das distale Ende des Katheters.

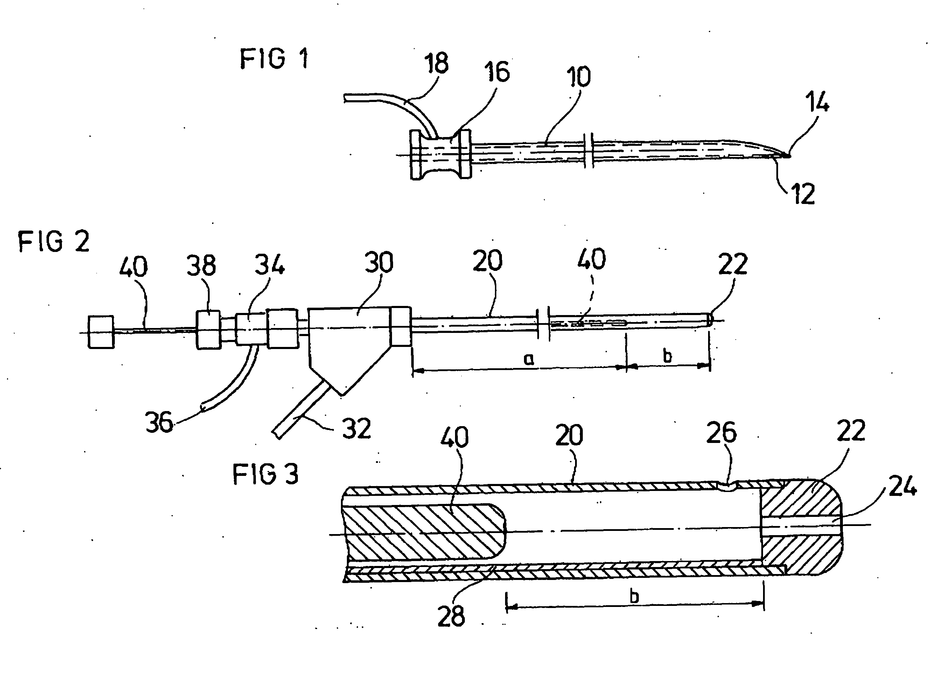

Das gesamte Set besteht aus einer in Figur 1 gezeigten Kanüle 10 und dem in Figur 2 gezeigten eigentlichen Katheterset.

Die Kanüle 10 ist vorzugsweise als unipolare Stimulationskanüle ausgebildet, die aus einem starren Stahlrohr besteht, welches an seinem distalen Ende im dargestellten Beispiel als Tuohy-Spitze 12 ausgebildet ist. Selbstverständlich kann die Spitze auch mit einem Facetten-Schliff oder als Sprotte-Spitze ausgebildet sein. Die Kanüle 10 ist über ihre gesamte Länge mit einer äußeren isolierenden Beschichtung versehen, die lediglich eine punktförmige Spitze 14 freilässt. Am proximalen Ende der Kanüle 10 ist ein Kunststoffanschluss 16 angebracht, durch welchen eine Anschlusslitze 18 geführt ist, die das metallische Kanülenrohr kontaktiert und zum Anschluss eines elektrischen Stimulators dient. Durch den Anschluss 16 kann der nachfolgend beschriebene Katheter koaxial in das Rohr der Kanüle 10 eingeschoben werden.

Der in den Figuren 2 und 3 dargestellte Katheter 20 ist ein flexibler Katheter aus einem nicht leitenden weichen Kunststoff, dessen Länge je nach Anwendungsfall zwischen 15 cm und 100 cm betragen kann. Gegebenenfalls kann die Wandung des Katheters 20 auch durch eine eingelegte oder eingebettete Drahtwendel verstärkt sein, um ein Abknicken oder Zusammenquetschen des Katheters 20 zu erschweren. Die flexible Biegsamkeit des Katheters wird hierdurch nicht beeinträchtigt. Der Katheter 20 weist an seinem distalen Ende eine Stimulationsspitze 22 auf, die in dem dargestellten Ausführungsbeispiel eine elektrisch leitende Kappe ist, die in das distale Ende des Katheters 20 eingesetzt ist. Am distalen Ende des Katheters 20 ist wenigstens eine Austrittsöffnung für eine über den Katheter zugeführte Flüssigkeit vorgesehen. Im dargestellten Ausführungsbeispiel ist eine Austrittsöffnung 24 als koaxiale Öffnung ausgebildet, die durch die Stimulationsspitze 22 hindurch führt. Eine weitere Austrittsöffnung 26 ist unmittelbar hinter der Stimulationsspitze 22 in der Wandung des Katheters 20 vorgesehen. Die Austrittsöffnungen 24 und 26 können alternativ oder auch gemeinsam vorgesehen sein. In dem Katheter 20 verläuft eine elektrisch leitende Verbindung, die im dargestellten Ausführungsbeispiel als dünner Draht 28 ausgebildet ist. Dieser Draht 28 ist distal mit der Stimulationsspitze 22 leitend verbunden und führt über die gesamte Länge des Katheters 20 zu dessen proximalem Ende. Am proximalen Ende des Katheters 20 ist ein Anschlussadapter 30 aus Kunststoff angebracht. Der Anschlussadapter 30 nimmt einen Stimulationsanschluss 32 auf, der als Litze seitlich in den Anschlussadapter 30 eingeführt ist und beim Einspannen des Katheters 20 in den Anschlussadapter 30 den Draht 28 kontaktiert. Der Stimulationsanschluss 32 dient zum Anschluss eines elektrischen Stimulators.

Koaxial in das proximale Ende des Anschlussadapters 30 ist ein Schlauchadapter 34 einsetzbar. Ein Zuspritzschlauch 36 führt seitlich in den Schlauchadapter 34. An den Zuspritzschlauch 36 ist eine nicht dargestellte Spritze anschließbar. über den Zuspritzschlauch 36 und den Schlauchadapter 34 kann eine Flüssigkeit in den Katheter 20 zugeführt oder eine Flüssigkeit über den Katheter 20 aspiriert werden. Hierzu weist der Schlauchadapter 34 ein hämostatisches Ventil auf. Der Schlauchadapter 34 kann an seinem proximalen Ende durch einen Verschluss 38 verschlossen werden, um ein Injizieren oder Aspirieren über den Schlauchadapter 34 und den Zuspritzschlauch 36 zu ermöglichen.

Der Anschlussadapter 30 und der Schlauchadapter 34 bilden einen axial durchgehenden und axial mit dem Katheter 20 fluchtenden Kanal. Durch diesen Kanal kann ein Versteifungsmandrin 40 vom proximalen Ende her durch den Schlauchadapter 34 und den Anschlussadapter 30 in den Katheter 20 eingeschoben werden. Die Länge des Versteifungsmandrins 40 ist so bestimmt, dass dieser, wenn er vollständig in den Katheter 20 eingeschoben ist, mit seinem distalen Ende bis an das innere distale Ende des Katheters 20 reicht, im dargestellten Beispiel an das innere Ende der Stimulationsspitze 22. Der Versteifungsmandrin 40 ist ein steifer Stahldraht, der elastisch biegsam aber knickfest ist. Der Durchmesser des Versteifungsmandrins 40 ist etwas kleiner als der Innendurchmesser des Katheters 20, sodass zwischen der äußeren Mantelfläche des Versteifungsmandrins 40 und der inneren Mantelfläche des Katheters 20 ein Ringspalt verbleibt, über welchen durch den Zuspritzschlauch 36 zugeführte Flüssigkeit zur distalen Katheterspitze gelangen kann.

Der Versteifungsmandrin 40 kann in dem Katheter 20 wählbar weit nach distal vorgeschoben werden. In dem axialen Längenbereich a des Katheters, in welchem sich der Versteifungsmandrin 40 hierbei befindet, wird der weich-biegsame Katheter 20 durch den Versteifungsmandrin 40 versteift. In dem distalen Endbereich b des Katheters 20, in welchen der Versteifungsmandrin 40 nicht geschoben ist, behält der Katheter 20 seine weichflexible Eigenschaft und kann Hindernissen ohne merklichen Widerstand ausweichen. Durch axiales Verschieben des Versteifungsmandrins 40 kann die Länge dieses weichen Spitzenbereichs b des Katheters 20 den anatomischen Gegebenheiten angepasst werden, durch welchen die Katheterspitze jeweils aktuell vorgeschoben werden muss.

Das verfahren zur epiduralen oder peripheren Nervenblockade verläuft in folgenden Schritten:

Zunächst wird die Kanüle 10 in den Wirbelkanal und den Epiduralraum (bei der epiduralen Nervenblockade) oder in die Nervenscheide (bei der peripheren Nervenblockade) eingestochen. Über die Anschlusslitze 18 wird die Kanüle 10 an einen elektrischen Stimulator angeschlossen, der Stimulationspulse über die Spitze 14 an das jeweils an die Spitze 14 angrenzende Nervengewebe abgibt. Durch diese Nervenstimulation kann die exakte Lage der Kanülenspitze 12 lokalisiert und positioniert werden.

Nachdem die Kanüle 10 mit ihrer Spitze 12 positioniert ist, wird der Katheter 20 durch den Anschluss 16 in die Kanüle 10 eingeführt. Der Katheter 20 tritt mit seiner distalen Spitze aus dem distalen Ende der Kanüle 10 aus und wird in den Epiduralraum bzw. der Nervenscheide vorgeschoben. In den Katheter 20 ist dabei der Versteifungsmandrin 40 eingeschoben.

Während des Vorschiebens des Katheters 20 wird der Stimulationsanschluss 32 an den elektrischen Stimulator angeschlossen und Stimulationspulse werden über den Stimulationsanschluss 32 und den Draht 28 zu der Stimulationsspitze 22 geleitet, sodass die jeweilige Position der distalen Spitze des Katheters 20 über die Nervenstimulation mittels der Stimulationsspitze 22 lokalisiert werden kann. Je nach den anatomischen Gegebenheiten des Bereichs, durch welchen die distale Spitze des Katheters 20 aktuell vorgeschoben wird, kann der Versteifungsmandrin 40 mehr oder weniger weit axial in dem Katheter 20 distal nach vorn geschoben werden. Dadurch kann der Anästhesist die Steifigkeit bzw. die weiche Nachgiebigkeit der distalen Spitze des Katheters 20 beeinflussen und den anatomischen Gegebenheiten optimal anpassen.

Während des Vorschiebens des Katheters 20 kann über den Zuspritzschlauch 36 eine Flüssigkeit in den Katheter 20 eingeleitet werden, die über die Austrittsöffnungen 24 bzw. 26 austritt. Diese Flüssigkeit kann dazu dienen, den Raum, durch welchen die distale Spitze des Katheters 20 aktuell vorgeschoben wird, zu dilatieren und ein flüssiges Polster zu bilden, welches das Vorschieben der Katheterspitze erleichtert. Hierzu kann bspw. eine wässerige Dextroselösung dienen. Es kann auch eine Kochsalzlösung zugespritzt werden, die aufgrund ihrer elektrischen Leitfähigkeit erforderlichenfalls den Kontakt zwischen der Stimulationsspitze 22 und dem umgebenden Nervengewebe verbessert.

Sobald das distale Ende des Katheters 20 mittels der Stimulationsspitze 22 in die gewünschte Position gebracht ist, wird der Versteifungsmandrin 40 aus dem Katheter 20 abgezogen und der Anschlussadapter 30 von dem Katheter 20 dekonnektiert. Nun kann die Kanüle 10 nach proximal von dem Katheter 20 abgezogen werden und der Katheter 20 kann zunächst für eine kontinuierliche Anästhesie oder für ein späteres Nachdosieren des Anästhetikums in seiner Lage verbleiben. Über die Stimulationsspitze 22 kann dabei erforderlichenfalls auch die Lage des Katheters 20 nachträglich korrigiert werden.

### Bezugszeichenliste

- 10: Kanüle
- 12: Tuohy-Spitze
- 14: Spitze
- 16: Anschluss
- 18: Anschlusslitze
- 20: Katheter
- 22: Stimulationsspitze
- 24: Austrittsöffnung
- 26: Austrittsöffnung
- 28: Draht
- 30: Anschlussadapter
- 32: Stimulationsanschluss
- 34: Schlauchadapter
- 36: Zuspritzschlauch
- 38: Verschluss
- 40: Versteifungsmandrin

## Patentansprüche

1. Katheterset für die epidurale oder periphere Nervenblockade, mit einer Kanüle (10), mit einem durch die Kanüle (10) einführbaren, weich nachgiebig flexiblen Katheter (20) aus einem weichen elektrisch isolierenden Kunststoff, mit wenigstens einer am distalen Ende des Katheters (20) angeordneten Austrittsöffnung (24, 26) für eine Flüssigkeit, mit einer am distalen Ende des Katheters (20) freiliegenden elektrisch leitenden Stimulationsspitze (22), mit einem am proximalen Ende des Katheters (20) angeordneten elektrischen Stimulationsanschluss (32) und mit einer im Inneren des Katheters (20) verlaufenden leitenden Verbindung (28) der Stimulationsspitze (22) mit dem Stimulationsanschluss (32),
**dadurch gekennzeichnet, dass**
in dem Katheter (20) ein Versteifungsmandrin (40) angeordnet ist, der in seiner axialen Lage in dem Katheter (20) in der Weise positionierbar ist, dass vor dem distalen Ende des Versteifungsmandrins (40) ein wählbar langer distaler flexibler Endbereich (b) des Katheters (20) verbleibt, dass am proximalen Ende des Katheters (20) ein Anschlussadapter (30) angeordnet ist, der den Stimulationsanschluss (32) aufnimmt und durch den koaxial der Versteifungsmandrin (40) einführbar ist, dass proximal an dem Anschlussadapter (30) ein Schlauchadapter (34) ansetzbar ist, der ein hämostatisches Ventil aufweist und in den ein Zuspritzschlauch (36) einmündet, dass der Versteifungsmandrin (40) koaxial durch das verschließbare proximale Ende des Schlauchadapters (34) einführbar ist, und dass der Durchmesser des Versteifungsmandrins (40) etwas kleiner als der Innendurchmesser des Katheters (20) ist, wodurch zwischen der äußeren Mantelfläche des Versteifungsmandrins (40) und der inneren Mantelfläche des Katheters (20) ein Ringspalt verbleibt, über welchen durch den Zuspritzschlauch (36) zugeführte Flüssigkeit zur distalen Katheterspitze gelangen kann.

2. Katheterset nach Anspruch 1,
**dadurch gekennzeichnet, dass**
das distale Ende des Katheters (20) zumindest bis auf eine eventuelle Austrittsöffnung (24) geschlossen ist.

3. Katheterset nach Anspruch 1,
**dadurch gekennzeichnet, dass**
die Kanüle (10) eine unipolare Stimulationskanüle ist.

## Claims

1. Catheter set for epidural or peripheral nerve block, comprising a cannula (10), a soft pliably flexible catheter (20) which is made from a soft electrically insulating plastic and which can be introduced through the cannula (10), at least one exit opening (24, 26) which is arranged at the distal end of the catheter (20) for a liquid, an electrically conductive stimulation tip (22) which is exposed at the distal end of the catheter (20), an electrical stimulation connection (32) which is arranged at the proximal end of the catheter (20), and a conductive connection (28) of the stimulation tip (22) to the stimulation connection (32), said connection (28) running inside the catheter (20), **characterised in that** arranged in the catheter (20) is a stiffening stylet (40) which can be positioned in terms of its axial position in the catheter (20) in such a way that a distal flexible end region (b) of the catheter (20), of selectable length, remains in front of the distal end of the stiffening stylet (40), **in that** arranged at the proximal end of the catheter (20) is a connection adapter (30) which receives the stimulation connection (32) and through which the stiffening stylet (40) can be introduced coaxially, **in that** there can be placed proximally on the connection adapter (30) a tube adapter (34) which has a haemostatic valve and into which an injection tube (36) opens, **in that** the stiffening stylet (40) can be introduced coaxially through the closable proximal end of the tube adapter (34), and **in that** the diameter of the stiffening stylet (40) is somewhat smaller than the inner diameter of the catheter (20), as a result of which an annular gap remains between the outer surface of the stiffening stylet (40) and the inner surface of the catheter (20), via which annular gap it is possible for liquid supplied through the injection tube (36) to reach the distal catheter tip.

2. Catheter set according to claim 1, **characterised in that** the distal end of the catheter (20) is closed at least as far as a possible exit opening (24).

3. Catheter set according to claim 1, **characterised in that** the cannula (10) is a unipolar stimulation cannula.

## Revendications

1. Appareillage de cathéter de blocage des nerfs épiduraux ou périphériques comprenant :
- une canule (10),
- un cathéter (20) souple, élastique, s'introduisant à travers la canule (10), ce cathéter étant en une matière plastique souple isolante élastique,
- l'extrémité distale du cathéter (20) ayant au moins un orifice de sortie (24, 26) pour un liquide,
- une aiguille de stimulation (22) électroconductrice, étant située librement à l'extrémité distale du cathéter (20),
- l'extrémité proximale du cathéter (20) ayant un branchement de stimulation électrique (32), et
- une liaison électrique (28) passant à l'intérieur du cathéter (20) pour relier l'aiguille de stimulation (22) au branchement de stimulation (32),
**caractérisé en ce que**
- un mandrin de rigidification (40) est logé dans le cathéter (20), ce mandrin pouvant être positionné axialement dans le cathéter (20) de façon à laisser devant l'extrémité distale du mandrin de rigidification (40), une zone d'extrémité (b) du cathéter (20), de longueur distale souple, choisie,
- l'extrémité proximale du cathéter (20) comporte un adaptateur de branchement (30) recevant le raccord de stimulation (32) par lequel s'introduit coaxialement le mandrin de rigidification (40),
- en position proximale, l'adaptateur de raccordement (30) comporte un adaptateur de tuyau (34) muni d'une vanne hémostatique, et dans lequel débouche un tuyau d'injection (36),
- le mandrin de rigidification (40) s'introduit coaxialement à travers l'extrémité proximale susceptible d'être fermée de l'adaptateur de tuyau (34), et
- le diamètre du mandrin de rigidification (40) est légèrement inférieur au diamètre intérieur du cathéter (20) pour laisser entre la surface enveloppe extérieure du mandrin de rigidification (40) et la surface enveloppe intérieure du cathéter (20), un intervalle annulaire à travers lequel le liquide du tuyau d'injection (36) peut arriver jusqu'à la pointe distale du cathéter.

2. Appareillage de cathéter selon la revendication 1,
**caractérisé en ce que**
l'extrémité distale du cathéter (20) est fermée au moins jusqu'à laisser un éventuel orifice de sortie (24).

3. Appareillage de cathéter selon la revendication 1,
**caractérisé en ce que**
la canule (10) est une canule de stimulation unipolaire.
